# EUROPEAN PATENT SPECIFICATION

(11) **EP 1 954 803 B1**
(45) Date of publication and mention of the grant of the patent: **22.05.2013**
(21) Application number: 06812485.8
(22) Date of filing: 07.11.2006
(51) Int. Cl.: C12N 5/074

(54) **MULTIPOTENT ADULT STEM CELLS HAVING AN ABILITY OF OCT4 EXPRESSION DERIVED FROM UMBILICAL CORD BLOOD AND METHOD FOR PREPARING THE SAME**
AUS NABELSCHNURBLUT STAMMENDE MULTIPOTENTE STAMMZELLEN MIT FÄHIGKEIT ZUR OCT4-EXPRESSION UND VERFAHREN ZUR PRÄPARATION DAVON
CELLULES SOUCHES ADULTES MULTIPOTENTES POUVANT EXPRIMER OCT4 DERIVEES DU SANG DU CORDON OMBILICAL ET PROCEDE DE PREPARATION CORRESPONDANT

(30) Priority: 02.12.2005 KR 20050117015
(43) Date of publication of application: 13.08.2008
(73) Proprietor: SEOUL NATIONAL UNIVERSITY INDUSTRY FOUNDATION, Gwanak-gu Seoul 151-050 (KR)
(72) Inventor: KANG, Kyung Sun, Seoul 137-935 (KR)
(74) Representative: Hiebl, Inge Elisabeth
(86) International application number: PCT/KR2006/004649
(87) International publication number: WO 2007/064090

(56) References cited:
- WO-A-02/064748
- WO-A-2005/113754
- WO-A-2007/024036
- US-A1- 2004 107 453
- US-A1- 2006 182 724
- DA SILVA C.L. ET AL.: 'A human stromal-based serum-free culture system supports the ex vivo expansion/maintenance of bone marrow and cord blood hematopoietic stem/progenitor cells' EXP. HEMATOL. vol. 33, no. 7, July 2005, pages 828 - 835, XP004936584
- KIM S.Y. ET AL.: 'Differentiation of endothelial cells from human umbilical cord blood AC133-CD14+ cells' ANN. HEMATOL. vol. 84, no. 7, July 2005, pages 417 - 422, XP019333926
- TONDREAU T. ET AL.: 'Mesenchymal Stem Cells Derived from CD133-Positive Cells in Mobilized Peripheral Blood and Cord Blood: Proliferation, Oct4 Expression, and Plasticity' STEM CELLS vol. 23, no. 8, September 2005, pages 1105 - 1112, XP003013395
- KASHIWAKURA I. ET AL.: 'Basic fibroblast growth factor-stimulated ex vivo expansion of haematopoietic progenitor cells from human plancetal and umbilical cord blood' BR. J. HAEMATOL. vol. 122, no. 3, August 2003, pages 479 - 488, XP003013396

## Description

### TECHNICAL FIELD

The present disclosure relates to multipotent adult stem cells expressing Oct4 from umbilical cord blood, and more specifically, to a method for differentiating multipotent adult stem cells into osteogenic cells or into nenre cells which are obtained by culturing umbilical cord blood-derived monocyte in a medium containing bFGF (basic fibroblast growth factor) and human serum or plasma to isolate. '

### BACKGROUND ART

Although many diseases of the past have been cured in our modern society due to the development of life sciences and medical sciences, there still have been inveterate diseases or incurable diseases such as ischemic necrosis, cancer, dementia and a severe burn, and the like. Moreover, there are many problems in the filed of organ transplantation. As a therapy to treat the root cause of these diseases, cell therapy has been in the limelight these days.

Cell therapy is a method for treating or preventing diseases by externally proliferating or sorting autologous stem cells, allogenic stem cells or xenogenic stem cells, or other methods such as changing biological characteristics of cells. Cell therapy has infinite possibilities in the treatment of inveterate diseases and incurable diseases since it has a very wide range of application areas, for example, proliferating somatic cells collected from the patient himself, other person or other animals or differentiating stem cells into desired cell types to use in the treatment of diseases.

It has been reported that when skeletal myoblasts, endothelial progenitor cells or bone marrow stem cells are transplanted into infracted myocardium, myocardial function is improved (Menasche, P. et al., J. Am. Coll. Cardiol., 41:1078, 2003; Strauer, B.E. et al., Circulation, 106:1913, 2002; and Stamm, C. et al., Lancet, 361:45, 2003). Such improvement of myocardial function is due to formation of mechanical scaffold by arteriogenic cytokine secreted by stem cells or other beneficial cells gathering into ischemic region (Orlic, D. et al., Circ Res, 91:1092, 2002).

Meanwhile, the term "stem cells" refers to cells having not only self-replication ability but also the ability to differentiate into different cell types, and can be divided into totipotent stem cells, pluripotent stem cells, and multipotent stem cells.

Totipotent stem cells are cells having totipotential differentiation properties, which are capable of developing into a complete organism, and the property is possessed by cells up to the 8-cell stage after fertilization of the oocyte by the sperm. When these cells are isolated and transplanted into the uterus, they can develop into a complete organism.

Pluripotent stem cells are cells capable of developing into various cells and tissues derived from the ectodermal, mesodermal and endodermal layers, which are derived from the inner cell mass located inside of blastocysts, generated 4-5 days after fertilization. These cells are called "embryonic stem cells" and can differentiate into various other tissue cells but cannot form new living organisms.

Multipotent stem cells are stem cells differentiating normally into only cell types specific to their tissue and organ of origin, which are involved not only in the growth and development of various tissues and organs during the fetal, neonatal and adult periods but also in the maintenance of adult tissue homeostasis and the function of inducing regeneration upon tissue damage. Tissue-specific multipotent cells are collectively called "adult stem cells".

Umbilical cord blood-derived adult stem cells known up until now include mesenchymal stem cells capable of differentiating into osteocytes or skeletal muscles (Lee, O.K. et al., Blood, 103:1669, 2004; Gang, E.J. et al., BBRC, 321:102, 2004; Gang, E.J. et al., Stem Cell, 22:617, 2004), heart stem cells capable of differentiating into heart cells (US 2004/0126879) and endothelial progenitor cells (Yamamoto, K. et al., Arterio. Thromb. Vasc. Biol., 24:192, 2004).

WO 2007/024036 A1 discloses adult stem cells obtained by culturing umbilical cord-derived blood in a medium containing 5-20% of human serum or plasma, wherein the adult stem cells have specific properties with respect to their surface markers, growth and morphology. Further disclosed is a method of producing said adult stem cells and a cellular therapeutic agent containing said adult stem cells.

WO 2005/113754 discloses pluripotent adult stem cells and uses thereof.

WO 02/064748 discloses mammalian multipotent adult stem cells, and specifically method for obtaining, maintaining and differentiating said multipotent adult stem cells.

Especially, it has been known that lots of adult stem cells are contained in bone marrow, and the adult stem cells differentiate into only bone, cartilage, fat, and so on, because it is derived from mesoderm in embryonic stage. However, it was found that the adult stem cells can also differentiate into nerve cells which are derived from ectoderm, and thus, it is expected to have a very wide range of application areas in the treatment of diseases.

However, because the stem cells are capable of differentiating into a limited variety of cell types, including differentiation into osteocytes or skeletal muscles by mesenchymal stem cells, differentiation into heart cells by heart stem cells, and differentiation into vascular endothelial cells by endothelial progenitor cells, it is difficult to define these cells as true multipotent stem cells.

Accordingly, the present inventors have made an effort to obtain adult stem cells by a method comprising culturing umbilical cord blood-derived monocyte in a medium containing bFGF and human serum or plasma to isolate, and as a result, found that the method provides adult stem cells with excellent adhesion ability, whichexpress Oct4 which is a specific marker of embryonic stem cells, and which differentiate into various tissues such as osteogenic cells and nerve cells, thereby completing the present disclosure.

### SUMMARY OF THE INVENTION

It is an object of the present invention to provide a method for preparing adult stem cells, the method comprising the steps of culturing umbilical cord blood-derived monocyte in a medium being α-MEM containing bFGF and human serum or plasma, and recovering the adult stem cells, wherein the adult stem cells show the following characteristics:
(a) expressing Oct4;
(b) showing positive immunological responses to all of CD24, CD29, CD31, CD 44, CD33, CD45 and CD49B, and negative immunological responses to CD34, CD51/61, CD62L, CD62P, CD90, CD133 and CD135;
(c) growing on plastics and showing round-shaped or spindle-shaped morphological features; and
(d) having the ability to differentiate into the cells derived from mesoderm, endoderm and ectoderm.

In the present invention, the content of bFGF and human serum or plasma is preferably 1∼30ng/mℓ and 5∼30%, respectively, and the human serum or plasma is autologous or allologous, and the medium is α-MEM.

The present disclosure also provides a method for differentiating adult stem cells into osteogenic cells, comprising the steps:
(a) preparing adult stem cells according to the method comprising the steps of culturing umbilical cord blood-derived monocytes in a medium being α-MEM containing 10 ng/ml bFGF and 5-30% human serum or plasma, and recovering the adult stem cells; and
(b) culturing the oells obtained in step (a) in a medium comprising 0.1 µmol/L dexamethasone, 0.05mmol/L ascorbic acid-2-phosphate, 10mmol/L beta-glycophosphate and 5-30% human serum or plasma in condition of 5% CO₂ at 37°C.

The present disclosure further provides a method for differentiating adult stem cells into nerve cells, comprising the steps:
(a) preparing adult stem cells according to the method comprising the steps of culturing umbilical cord blood-derived monocytes in a medium being α-MEM containing 10 ng/ml bFGF and 5-30% human serum or plasma, and recovering the adult stem cells; and
(b) culturing the cells obtained in step (a) in a medium comprising 10ng/ml bFGF, 10ng/ml human epidermal growth factor, 10ng/ml human neural growth factor and 5-30% human serum or plasma in condition of 5% CO₂ at 37°C. The cells derived from mesoderm are preferably osteogenic cells or endothelial cells, and the cells derived from ectoderm are preferably nerve cells.

Another features and embodiments of the present invention will be more clarified from the following "detailed description" and the appended "claims".

### BRIEF DESCRIPTION OF DRAWINGS

FIG. 1 is a photograph of umbilical cord blood-derived multipotent adult stem cells according to the present disclosure.

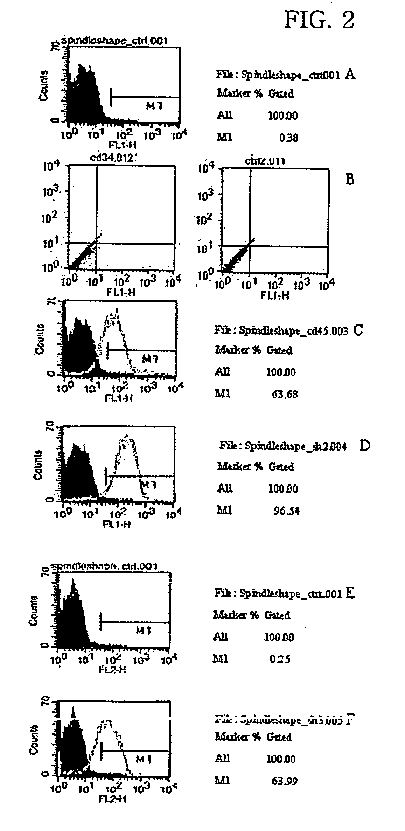

FIG. 2 shows the immunological characteristics of umbilical cord blood-derived multipotent stem cells, measured by flow cytometry. A and E: control groups; B: CD34; C: CD45; D: SH-2; and F: SH-3.
FIG. 3 shows the results of PAS staining conducted to examine the expression or non-expression of antigens in multipotent adult stem cells according to the present disclosure.
FIG. 4 is photographs of the multipotent adult stem cells derived from umbilical cord blood, differentiated into osteogenic cells(A and B), and the results of Von-Kossa staining (C and D).
FIG. 5 shows binding between umbilical cord blood-derived multipotent adult stem cells and specific antigens. A and B illustrate that the multipotent stem cells show positive responses to NSE (neuron-specific enolse), a neuron-specific antigen, and GFAP (glial fibrillary acidic protein), an astrocyte-specific antigen, respectively, and C and D show control groups.
FIG. 6 shows Oct4 expression in umbilical cord blood-derived multipotent stem cells after immunostained [A:phase contrast; B:Oct4; C:merge].
FIG. 7 shows the expression of SH-4 and Oct4 in umbilical cord blood-derived multipotent stem cells after immunostained [A:SH-4; B:Oct4; C:merge].

### DETAILED DESCRIPTION OF THE INVENTION, AND PPRFERRED EMBODIMENTS

The present invention relates to a method for preparing multipotent adult stem cells expressing Oct4 from umbilical cord blood, the preparation method is as follows.

At first, 70∼100mℓ of umbilical cord blood sample was diluted in PBS at a ratio of 1:1 to stir, and separated on ficoll at a Ficoll pague-umbilical cord blood ratio of 15:25. For this purpose, on 15 ml of a ficoll solution, the above blood sample solution (diluted in PBS at a ratio of 1:1) was centrifuged to obtain a white layer (monocytic layer), and the white layer was centrifuged. Herein, upper layer was completely removed and precipitation was stored in ice.

Next, the precipitation was shaken smoothly to disperse, and added with 10mℓ of culture broth to disperse uniformly, and then centrifuged at 1200rpm for 5 min, from which upper layer is completely removed and added with 10mℓ of culture broth to centrifuge at 1000rpm for 5 min (this process is repeated two times), thus obtaining cells.

α-MEM(Gibco) containing 5∼30% of human serum or plasma and 10ng/mℓ of bFGF (basic fibroblast growth factor)[Roche, Seitzerland] was added to the obtained cells, and optimum number of cells (about 3∼5 x 10⁶/mℓ) were plated in a culture vessel to culture for 3 days. After 3 days, relatively heavy cells such as RBC(red blood cell) exist in the lower layer of culture broth, and adult stem cells to be isolated exist in the upper layer. The upper layer is carefully collected to move into a new culture vessel to culture, thus finally obtaining adult stem cells having the ability to express Oct4.

Methods for obtaining multipotent stem cells expressing desired surface antigens from the obtained stem cell broth include FACS method using flow cytometer with sorting function *(*Int. Immunol., 10(3):275, 1998), a method using magnetic beads, and panning method using an antibody specifically recognizing multipotent stem cells *(*J. Immunol., 141(8):2797, 1998). Also, methods for obtaining multipotent stem cells from a large amount of culture broth include a method where antibodies, which is expressed on the surface of cells to specifically recognize molecules (hereinafter, referred to as "surface antigens"), are used alone or in combination as columns.

Flow cytometric sorting methods may include droplet charge method and cell capture method. In any of these methods, an antibody specifically recognizing an antigen on the cell surface is fluorescently labeled, the intensity of fluorescence emitted from the antibody bonded with the molecule expressed on the surface of the cell is converted to an electric signal whereby the amount of antigen expressed on cells can be quantified. It is also possible to separate cells expressing a plurality of surface antigens by combination of fluorescence types used therefore. Examples of fluorescent labels which can be used in this case include FITC (fluorescein isothiocyanate), PE (phycoerythrin), APC (allo-phycocyanin), TR (Texas Red), Cy 3, CyChrome, Red 613, Red 670, TRI-Color, Quantum Red, etc.

FACS methods using flow cytometer include: a method where the obtained stem cell broth is collected, from which cells are isolated by, for example, centrifugation, and stained directly with antibodies; and a method where the cells are cultured and grown in a suitable medium and then stained with antibodies. The staining of cells is performed by mixing a primary antibody recognizing a surface antigen with a target cell sample and incubating the mixture on ice for 30 minutes to 1 hour. When the primary antibody is fluorescently labeled, the cells are isolated with a flow cytometer after washing. When the primary antibody is not fluorescently labeled, cells reacted with the primary antibody and a fluorescently-labeled secondary antibody having binding activity to the primary antibody are mixed after washing, and incubated on ice water for 30 minutes to 1 hour. After washing, the cells stained with the primary and secondary antibodies are isolated with a flow cytometer.

The method using magnetic beads allows mass isolation of cells expressing the target surface antigens. Although cell purity by this isolation method is lower than that by the above-described method using the flow cytometer, it can secure sufficient cell purity by repeated purification.

As a marker, there may be an embryonic stem cell-specific protein, hematopoietic antigens, surface antigens of mesenchymal cells, and neuron-specific antigens of the nervous system, and the like. The embryonic stem cell-specific proteins include Oct4, and the like and the hematopoietic antigens include CD34 and CD45, and the like and the surface antigens of mesenchymal cells include SH-2 and SH-3, and the like and the neuron-specific antigens of the nervous system include NSE and GFAP, and the like. The single or combined use of antibodies recognizing the above-described surface antigens enables desired cells to be obtained.

The multipotent adult stem cells show positive response to monocyte-macrophage antigen CD45, negative response to hematopoietic lineage antigen CD34 and express Oct4, thus it is assumed to be stem cells in which differentiation into monocytes from hematopoietic cells was proceeding.

### Examples

Hereinafter, the present invention will be described in more detail by examples.

### Example 1: Isolation of adult stem cells from umbilical cord blood

Umbilical cord blood was collected from full-term and preterm newborns in Seoul National University Hospital and Samsung Cheil Hospital according to Institutional Review Board guidelines.

70-100 ml of the collected umbilical cord blood sample was diluted in PBS at a ratio of 1:1 to stir. Then, the blood sample was separated on ficoll at a ratio of 15:25, in which the blood sample (diluted in PBS at a ratio of 1:1) was spilled smoothly onto 15 ml of Ficoll solution to cause layer separation, followed by centrifugation at 2500 rpm for 20 minutes. After the centrifugation, three different layers were formed starting from the bottom, among them, buffy coat (the middle layer:monocytic layer) was taken with a micropipette, and washed three times with HBSS, followed by centrifugation at 1800 rpm for 15 minutes, from which upper layer is completely removed and a precipitation was stored in ice.

The precipitation was shaken smoothly to disperse, and added with 10mℓ of culture broth to disperse uniformly, and then centrifuged at 1200rpm for 5 min, from which upper was completely removed and added with 10mℓ of culture broth to centrifuge at 1000rpm for 5 min (this process is repeated two times), thus obtaining cells.

α-MEM(Gibco) containing 5∼30% of human serum or plasma and 10ng/mℓ of bFGF (basic fibroblast growth factor)[Roche, Seitzerland] was added to the obtained cells, and optimum number of cells (about 3∼5 x 10⁶/mℓ) were plated in a culture vessel to culture for 3 days. After 3 days, relatively heavy cells such as RBC (red blood cell) existed in the lower layer of culture broth and adult stem cells to be isolated existed in the upper layer, from which the upper layer is carefully separated to move into a new culture vessel to culture, thus finally obtaining adult stem cells.

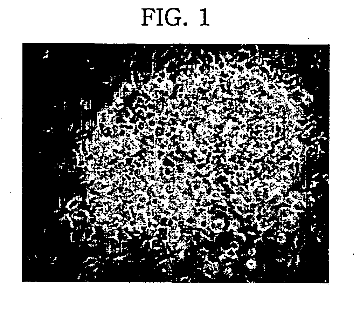

Fig. 1 is microphotograph of multipotent adult stem cells derived from umbilical cord blood according to the present disclosure.

### Example 2: Immunological characteristics of umbilical cord blood-derived multipotent adult stem cells

To examine the immunological characteristics of the umbilical cord blood-derived multipotent adult stem cells obtained in Example 1, the pattern of cell surface antigen expression was analyzed. For this purpose, 2x10⁶-10⁷ of the cells cultured in Example 1 were washed with PBS solution and incubated with their corresponding antibodies at room temperature. The expression or non-expression of the antigens was analyzed with a flow cytometer. Also, PAS staining periodic acid Schiff staining) was conducted.

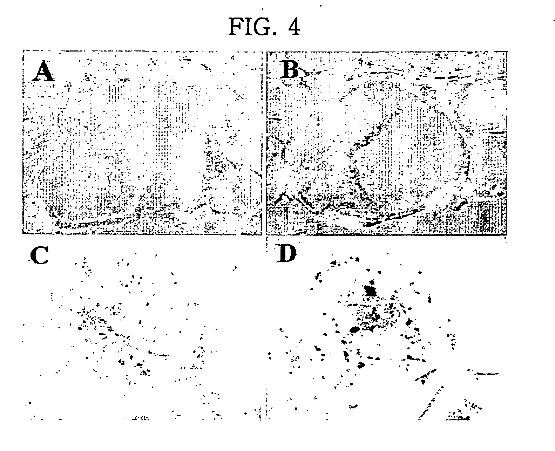

As a result, as shown in FIG. 4, the umbilical cord blood-derived multipotent adult stem cells showed 63.38%, 96.54% and 63.99% of positive responses to CD45, SH-2 and SH-3, respectively, and more than 90% of negative response to CD34. Also, immunophenotypes of other antigens were analyzed and as a result, the immunological characteristics of the multipotent adult stem cells were all negative for CD51/61, CD62L, CD62P, CD133, CD135, CD90, positive for CD29, positive for CD44, positive for CD49B, positive or negative for CD105(SH-2), and negative for CD90.

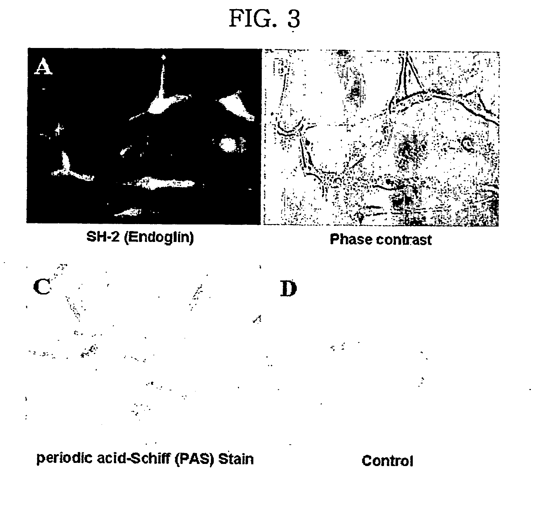

Meanwhile, as shown in FIG. 3, the multipotent adult stem cells showed positive response in PAS staining.

### Example 3: Differentiation of umbilical cord blood-derived multipotent adult stem cells into osteogenic cells

The umbilical cord blood-derived multipotent adult stem cell broth obtained in Example 1 was diluted in 1 ml of osteogenesis-inducing medium (0.1 µmol/L dexamethasone (Sigma, USA), 0.05 mmol/L ascorbic acid-2-phosphate (Sigma, USA), 10 mmol/L beta-glycophosphate (Sigma), and 5-30% human serum or plasma) to count the number of cells. Then, the cells were cultured in a flask (5% CO₂; 37 °C; medium replaced one time at 3-4-day intervals) to induce the differentiation of the multipotent adult stem cells into osteogenic cells. 14 days after the initiation of the culture, it was confirmed by Von-Kassa staining that the umbilical cord blood-derived multipotent adult stem cells differentiated into osteogenic cells (see FIG. 4).

### Example 4: Differentiation of umbilical cord blood-derived multipotent adult stem cells into nerve cells

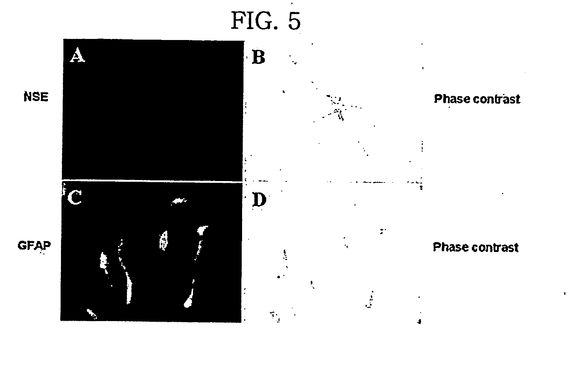

The umbilical cord blood-derived multipotent adult stem cell broth obtained in Example 1 were diluted in 1 ml of neural medium (containing 10 ng/ml basic fibroblast growth factor (Roche, Switzerland), 10 ng/ml human epidermal growth factor (Roche, Switzerland), 10ng/ml human neural growth factor (Invitrogen, USA) and 5-30% human serum or plasma). The cells were cultured on a flask in 5% CO₂ incubator at 37°C to induce differentiation of the multipotent adult stem cells into nerve cells. 14 days after the initiation of the culture, it was confirmed that the multipotent stem cells showed positive responses to NSE (neuron-specific enolase), a neuron-specific antigen, and GFAP (glial fibrillary acidic protein), an astrocyte-specific antigen. This suggests that the umbilical cord blood-derived multipotent adult stem cells differentiated into nerve cells (see FIG. 5).

### Example 5: Oct4 expression of umbilical cord blood-derived multipotent adult stem cells

The adult stem cells obtained in Example 1 were washed with PBS three times, added with 4% of paraformaldehyde solution and allowed to react for 5∼10 minutes at room temperature to fix the cells, followed by washing with PBS three times to remove the paraformaldehyde solution. The cells fixed by the above process were added with 0.3% of triton X-100 (in PBS) and allowed to react for 5 minutes at room temperature to make a condition where an exterior material is permeable into cells, followed by washing with PBS three times to remove the triton X-100.

The resulting cells were added with 10% of NGS, allowed to react for 30 minutes at room temperature, and added with PBS containing primary antibody (diluted at a ratio of 1:200) and NGS (diluted at a ratio of 1:100), and then allowed to react at 4 °C overnight, followed by washing with PBS three times to remove the reaction solution. After adding with PBS containing secondary antibody (diluted at a ratio of 1:200) and NGS (diluted at a ratio of 1:100), the resulting cells were allowed to react at 37 °C for 1hour to wash with PBS three times to remove the reaction solution, followed by mounting to observe with a microscope.

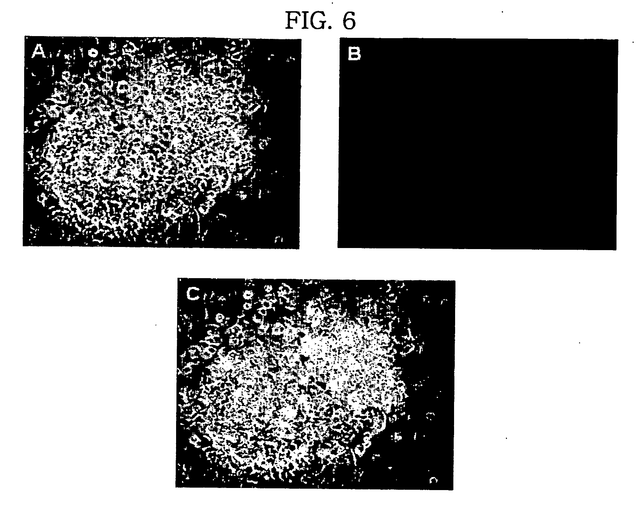

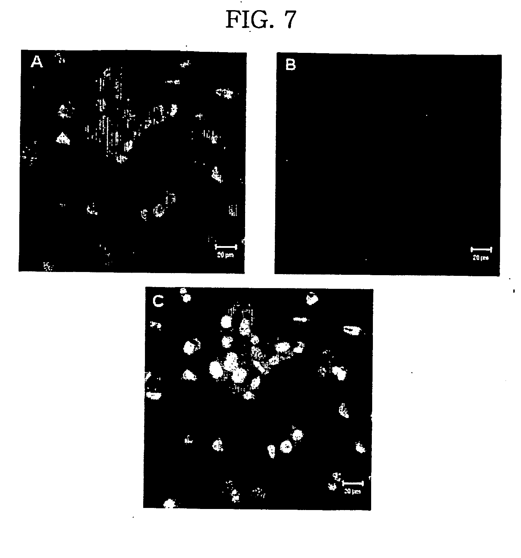

As a result, as shown in FIG 6, the adult stem **cells** prepared according to the method of the present : invention showed positive response to Oct4 which is a protein expressing embryonic stem cells, as well as a cell marker of undifferentiation. Furthermore, as shown in Fig 7, it is conformed that the expression of Oct4 and SH-4 was showed simultaneously in the adult stem cells according to the present disclosure.

Oct4 which is expressed in the adult stem cells according to the present disclosure is a transcription facter expressed in embryonic stem cells, which is involved in preventing cell differentiation, disappears after natural cell differentiation begins, and is known as a marker of pluripotent stem cells (Donovan, P.J., Nature Genet., 29:246, 2001; Pesce, M. and Scholer, H.R., Stem Cells, 19:271, 2001). Therefore, the result that the adult stem cells according to the present disclosure express the Oct4 which is a marker of pluripotent embryonic stem cells, clearly proves the fact that the adult stem cells according to the present disclosure is pluripotent.

### INDUSTRIAL APPLICABILITY

The present disclosure provides a method for preparing multipotent adult stem cells expressing Oct4, derived from umbilical cord blood using a medium containing bFGF and human serum or plasma. Although the stem cells according to the present disclosure are adult stem cells, they are multipotent and capable of differentiating according to another method of the present invention into osteogenic cells or nerve cells etc, thus they can be effectively used in the treatment of intractable disease and incurable disease..

## Claims

1. A method for preparing adult stem cells, the method comprising the steps of culturing umbilical cord blood-derived monocytes in a medium being α-MEM containing bFGF and human serum or plasma, and recovering the adult stem cells, wherein the adult stem cells show the following characteristics:
(a) expressing Oct4;
(b) showing positive immunological responses to all of CD24, CD29, CD31, CD33, CD45 and CD49B, and negative immunological responses to CD34, CD51/61, CD62L, CD62P, CD90, CD133 and CD135;
(c) growing on plastics and showing round-shaped or spindle-shaped morphological features; and
(d) having the ability to differentiate into the cells derived from mesoderm, endoderm and ectoderm.

2. The method for preparing adult stem cells according to claim 1, wherein the content of the bFGF and human serum or plasma is 1∼30ng/ml and 5∼30%, respectively.

3. The method for preparing adult stem cells according to claims 1, wherein the human serum or plasma is autologous or allologous.

4. A method for differentiating adult stem cells into osteogenic cells, comprising the steps:
(a) preparing adult stem cells according to the method of claim 1, said method comprising the steps of culturing umbilical cord blood-derived monocytes in a medium being α-MEM containing 10 ng/ml bFGF and 5-30% human serum or plasma, and recovering the adult stem cells; and
(b) culturing the cells obtained in step (a) in a medium comprising 0.1µmol/L dexamethasone, 0.05mmol/L ascorbic acid-2-phosphate, 10mmol/L beta-glycophosphate and 5-30% human serum or plasma in condition of 5% CO₂ at 37°C.

5. A method for differentiating adult stem cells into nerve cells, comprising the steps:
(a) preparing adult stem cells according to the method of claim 1, said method comprising the steps of culturing umbilical cord blood-derived monocytes in a medium being α-MEM containing 10 ng/ml bFGF and 5-30% human serum or plasma, and recovering the adult stem cells; and
(b) culturing the cells obtained in step (a) in a medium comprising 10ng/ml bFGF, 10ng/ml human epidermal growth factor, 10ng/ml human neural growth factor and 5-30% human serum or plasma in condition of 5% CO₂ at 37°C.

## Patentansprüche

1. Verfahren zum Herstellen adulter Stammzellen, wobei das Verfahren die Schritte des Kultivierens von Nabelschnurblut-abgeleiteten Monocyten in einem Medium, das α-MEM ist und bFGF und humanes Serum oder Plasma enthält, und das Gewinnen der adulten Stammzellen umfasst, wobei die adulten Stammzellen die folgenden Eigenschaften zeigen:
(a) exprimieren Oct4;
(b) zeigen positive immunologische Antworten auf allen CD24, CD29, CD31, CD33, CD45 und CD49B und negative immunologische Antworten auf CD34, CD51/61, CD62L, CD62P, CD90, CD133 und CD135,
(c) wachsen auf Kunststoffen und Zeigen runde oder spindelförmige morphologische Merkmale, und
(d) haben die Fähigkeit in die Zellen, die vom Mesoderm, Endoderm und Ektoderm abgeleitet sind, zu differenzieren.

2. Verfahren zum Herstellen adulter Stammzellen gemäß Anspruch 1, wobei die Menge des bFGF und humanen Serums oder Plasmas 1∼30 ng/ml bzw. 5∼30% ist.

3. Verfahren zum Herstellen adulter Stammzellen gemäß Anspruch 1, wobei das humane Serum oder Plasma autolog oder allolog ist.

4. Verfahren zum Differenzieren adulter Stammzellen in osteogene Zellen, umfassend die Schritte:
(a) Herstellen adulter Stammzellen gemäß dem Verfahren von Anspruch 1, wobei das Verfahren die Schritte des Kultivierens von Nabelschnurblut-abgeleiteten Monocyten in einem Medium, das α-MEM ist und 10 ng/ml bFGF und 5-30% humanes Serum oder Plasma enthält, und das Gewinnen der adulten Stammzellen umfasst, und
(b) Kultivieren der Zellen, die in Schritt (a) erhalten wurden, in einem Medium, das 0,1 µmol/L Dexamethason, 0,05 mmol/L Ascorbinsäure-2-phosphat, 10 mmol/L Beta-Glycophosphat und 5-30% humanes Serum oder Plasma umfasst, unter Bedingungen von 5% CO₂ bei 37°C.

5. Verfahren zum Differenzieren adulter Stammzellen in Nervenzellen, umfassend die Schritte:
(a) Herstellen adulter Stammzellen gemäß dem Verfahren von Anspruch 1, wobei das Verfahren die Schritte des Kultivierens von Nabelschnurblut-abgeleiteten Monocyten in einem Medium, das α-MEM ist und 10 ng/ml bFGF und 5-30% humanes Serum oder Plasma enthält, und das Gewinnen der adulten Stammzellen umfasst, und
(b) Kultivieren der Zellen, die in Schritt (a) erhalten wurden, in einem Medium, das 10 ng/ml bFGF, 10 ng/ml humanen epidermalen Wachstumsfaktor, 10 ng/ml humanen neuralen Wachstumsfaktor und 5-30% humanes Serum oder Plasma umfasst, unter Bedingungen von 5% CO₂ bei 37°C.

## Revendications

1. Procédé de préparation de cellules souches adultes, le procédé comprenant les étapes de culture de monocytes dérivés de sang du cordon ombilical dans un milieu qui est du α-MEM contenant le bFGF et du sérum ou du plasma humain, et de récupération des cellules souches adultes, dans lequel les cellules souches adultes présentent les caractéristiques suivantes :
(a) expression de Oct4 ;
(b) présentation de réponses immunologiques positives vis-à-vis de tout parmi CD24, CD29, CD31, CD33, CD45 et CD49B, et de réponses immunologiques négatives vis-à-vis de CD34, CD51/61, CD62L, CD62P, CD90, CD133 et CD135 ;
(c) croissance sur matière plastique et présentation des caractéristiques morphologiques de type forme arrondie ou en fuseau; et
(d) capacité à se différencier en les cellules dérivées du mésoderme, de l'endoderme et de l'ectoderme.

2. Procédé de préparation de cellules souches adultes selon la revendication 1, dans lequel la teneur en bFGF et en sérum ou plasma humain est respectivement de 1 à 30 ng/ml et 5 à 30 %.

3. Procédé de préparation de cellules souches adultes selon la revendication 1, dans lequel le sérum ou le plasma humain est autologue ou allologue.

4. Procédé de différenciation de cellules souches adultes en cellules ostéogéniques, comprenant les étapes :
(a) de préparation de cellules souches adultes selon le procédé selon la revendication 1, ledit procédé comprenant les étapes de culture de monocytes dérivés de sang du cordon ombilical dans un milieu qui est du α-MEM contenant 10 ng/ml de bFGF et 5 à 30 % de sérum ou de plasma humain, et de récupération des cellules souches adultes ; et
(b) de culture des cellules obtenues dans l'étape (a) dans un milieu comprenant 0,1 µmol/L de dexaméthasone, 0,05 mmol/L d'acide ascorbique-2-phosphate, 10 mmol/L de bêta-glycophosphate et 5 à 30 % de sérum ou de plasma humain sous des conditions de CO₂ à 5 % à 37°C.

5. Procédé de différenciation de cellules souches adultes dans des cellules nerveuses, comprenant les étapes :
(a) de préparation de cellules souches adultes selon le procédé selon la revendication 1, ledit procédé comprenant les étapes de culture de monocytes dérivés de sang du cordon ombilical dans un milieu qui est du α-MEM contenant 10 ng/ml de bFGF et 5 à 30 % de sérum ou de plasma humain, et de récupération des cellules souches adultes ; et
(b) de culture des cellules obtenues dans l'étape (a) dans un milieu comprenant 10 ng/ml de bFGF, 10 ng/ml de facteur de croissance épidermique humain, 10 ng/ml de facteur de croissance neuronale humain et 5 à 30 % de sérum ou de plasma humain sous des conditions de CO₂ à 5 % à 37°C.
